# EUROPÄISCHE PATENTSCHRIFT

(11) **EP 2 852 330 B1**
(45) Veröffentlichungstag und Bekanntmachung des Hinweises auf die Patenterteilung: **01.08.2018**
(21) Anmeldenummer: 13723793.9
(22) Anmeldetag: 21.05.2013
(51) Int. Cl.: A61B 17/04, A61B 17/00

(54) **FADEN-ANKER**
THREAD ANCHOR
ANCRAGE DE FILS

(30) Priorität: 21.05.2012 DE 202012005004 U
(43) Veröffentlichungstag der Anmeldung: 01.04.2015
(73) Patentinhaber: Stryker European Holdings I, LLC, Kalamazoo, MI 49002 (US); Woodwelding AG, 6362 Stansstad (CH)
(72) Erfinder: FRÉLY, Jean-Claude, 2504 Bienne (CH); KNECHT, Thomas, 5400 Baden (CH); BORDUSH, Astrid, Ottawa, Ontario K1E 3J2 (CA)
(74) Vertreter: Maiwald Patent- und Rechtsanwaltsgesellschaft mbH
(86) Internationale Anmeldenummer: PCT/EP2013/060349
(87) Internationale Veröffentlichungsnummer: WO 2013/174779

(56) Entgegenhaltungen:
- WO-A1-00/02489
- WO-A1-02/069817
- WO-A2-2008/034276
- US-A- 5 993 458

## Beschreibung

### GEBIET DER ERFINDUNG

Die Erfindung betrifft ein Verfahren und eine Vorrichtung zum Verankern eines Fadens an einem Objekt. Insbesondere betrifft die Erfindung ein Verfahren und eine Vorrichtung zum Verankern eines Fadens an einem Objekt unter Verwendung von Ultraschallschwingungen.

### HINTERGRUND DER ERFINDUNG

Um einen Faden an einem Objekt zu befestigen, insbesondere an einer Fläche eines Objekts, kann zum Beispiel mittels einer Schraube ein Faden zwischen Schraube und Objekt verklemmt werden. Diese Möglichkeit hat jedoch den Nachteil, dass der Faden nicht zuverlässig befestigt werden kann oder möglicherweise beschädigt wird.

Alternative ist bekannt, einen Faden vorab fest mit einer Schraube zu verbinden und dann diese Schraube in eine Bohrung in einem Objekt einzuschrauben. Die Handhabung einer derartigen Schrauben-Faden-Kombination ist jedoch umständlich, solange das freie Fadenende frei beweglich ist, während die Schraube eingeschraubt wird. Um diesen Nachteil zu überwinden, gibt es Systeme, bei welchen eine Schraube zusammen mit zumindest einem Faden lösbar verbunden mit einem geeigneten Werkzeug vor-konfektioniert bereitgestellt wird. Diese Variante ist jedoch teuer aufgrund des mit jeder Schraube mitzuliefernden Werkzeugs. Die WO 00/02489 offenbart ein Kit zum Verbinden eines oder mehrerer Fäden. Das Kit enthält einen Schmelzkragen und ein Fixierwerkzeug, das den Kragen um die Fäden herum komprimiert, so dass die Fäden sicher im Kragen gehalten werden und Teile des Kragens überlappen. Das Fixierwerkzeug bringt Energie auf den Kragen auf, um die überlappenden Teile des Kragens um die Fäden herum zu verschmelzen. Das Fixierwerkzeug besteht im Allgemeinen aus einer Energiequelle, einem Schweißkopf, einem Endeffektor und Elektronik zur Aktivierung des Endeffektors. Die US 5,993,458 beschreibt einen Knochenanker mit Außengewinde, der nach dem Einsetzen in einen Knochen in diesem bleiben kann und eine Plattform zur Aufnahme von Gewebe bilden kann, sowie ein Verfahren zur Ultraschall-Einbettung des Knochenankers in den Knochen mit und auch ohne Vorbohren eines Ankeraufnahmelochs. Der Knochenanker kann hierbei an der Spitze einer Ultraschall-Sonotrode befestigt werden und unter Verwendung von Ultraschallenergie in den Knochen eingeschraubt werden.

### ZUSAMMENFASSUNG DER ERFINDUNG

Es ist eine Aufgabe der Erfindung, ein Verfahren und eine Vorrichtung zum Verankern eines Fadens an einem Objekt bereitzustellen. Es kann als weitere Aufgaben angesehen werden, eine verbesserte Handhabung der Vorrichtung und eine zuverlässige und sichere Verankerung eines Fadens an einem Objekt zu gewährleisten.

Diese und weitere Aufgaben werden durch den jeweiligen Gegenstand der unabhängigen Ansprüche erfüllt. Weitere Ausführungsformen sind den jeweiligen abhängigen Ansprüchen zu entnehmen.

Im Allgemeinen weist eine Vorrichtung zum Verankern eines Fadens an einem Objekt einen Stift auf. Der Stift kann aus einem Material, beispielsweise einem geeigneten Polymer hergestellt sein, welches mittels Ultraschallschwingungen verflüssigbar ist. Ferner kann zumindest ein Faden mit dem Stift verbunden sein, wobei der zumindest eine Faden an einem hinteren Ende des Stifts befestigt sein kann. Der Stift kann beispielsweise einen Durchmesser von 2,8mm und eine Länge von 7mm haben.

Der Stift weist einen Körper mit einer Längsausdehnung (Länge) und einer Querausdehnung (Querschnitt, Durchmesser) auf, wobei der Stift vorzugsweise in Richtung der Längsausdehnung in eine Öffnung oder Aussparung in einem Objekt eingeführt bzw. eingesetzt werden kann. Zum leichteren Einführen kann das vordere Ende des Stifts verjüngt oder angeschrägt sein. Das hintere Ende des Stifts ist demnach dasjenige Ende, welches in Einführrichtung hinten an dem Stift ist.

Durch eine Fixierung des Fadens am hinteren Ende kann gewährleistet werden, dass der Faden beim Einführen des Stifts und damit beim Verankern nicht beschädigt wird. Es wird angemerkt, dass der Faden beispielsweise durch Eingießen beim Herstellen des Stiftes fest mit diesem verbunden werden kann, dass der Faden aber auch durch eine Öffnung (ein Querloch, ein Fadenauge) hindurch geführt oder mittels eines Hakens oder einer Klemmgabel an dem Stift fixiert werden kann.

Gemäß einer Ausführungsform weist die Vorrichtung ferner einen Träger mit einem Trägerkörper und zumindest einem Trägerarm auf, wobei der Trägerarm geeignet ist, den Stift in einer vorbestimmten Lage relative zum Trägerkörper lösbar zu halten.

Gemäß einer Ausführungsform kann der Träger ferner eine Fadenaufnahme zum Aufnehmen des Fadens aufweisen.

Mit Hilfe eines Trägers kann ein Stift zusammen mit einem Faden derart gehalten werden, dass der Stift (selbst wenn der Stift relative klein ist) zuverlässig in eine Bohrung in einem Objekt eingeführt werden kann, ohne dass es zu irgend einem Konflikt mit dem Faden kommt, welcher mit dem Stift verbunden ist.

Gemäß einer weiteren Ausführungsform weist die Vorrichtung ferner eine Schutzkappe auf, welche derart mit dem Träger verbindbar ist, dass ein von dem Trägerarm gehaltene Stift abgedeckt ist. Die Abdeckung des Stiftes durch eine Schutzkappe kann insbesondere im Bereich medizinischer Anwendungen vorteilhaft sein, da nicht nur Beschädigungen des Stiftes sondern auch Verschmutzungen beim Transport des Stiftes (am Träger) verhindert werden können.

Gemäß einer anderen Ausführungsform weist die Vorrichtung ferner einen Ultraschall-Applikator mit einer Sonotrode auf. Der Ultraschall-Applikator kann lösbar und derart mit dem Träger verbindbar sein, dass die Spitze der Sonotrode in Kontakt mit dem von dem Trägerarm gehaltenen Stift bringbar ist.

Mit anderen Worten, ein Träger mit Stift kann derart an einem Ultraschall-Applikator angeordnet werden, dass die Sonotrodenspitze in Kontakt mit dem hinteren Ende des Stifts ist. Damit kann der Stift, der von dem Träger gehalten wird, welcher wiederum mit dem Ultraschall-Applikator verbunden ist, sicher in eine Bohrung in einem Objekt eingeführt werden und mit Hilfe von Ultraschallschwingungen, die von dem Ultraschall-Applikator erzeugt werden können, verflüssigt oder zumindest weich gemacht werden, sodass das Material des Stiftes zuverlässig mit dem Material des Objektes verbunden werden kann bzw. an diesem verankert werden kann.

Gemäß einer Ausführungsform weist der Ultraschall-Applikator einen Riegel auf, wobei der Träger mittels des Riegels an dem Ultraschall-Applikator arretiert werden kann. Der Riegel kann darüber hinaus so gestaltet sein, dass auch eine Schutzkappe lösbar mit dem Ultraschall-Applikator verbunden werden kann.

Gemäß einer Ausführungsform weist der Träger der Vorrichtung zwei Trägerarme auf, die derart angeordnet sind, dass der Stift zwischen den zwei Trägerarmen gehalten werden kann, wobei die Trägerarme derart gestaltet sein können, dass auch die Spitze einer Sonotrode zwischen den Trägerarmen angeordnet werden kann. Ferner kann eine Fadenaufnahme an dem Trägerkörper vorgesehen sein. Wenn zwei Fäden mit dem Stift verbunden sind, kann jeweils ein Faden entlang eines Trägerarms zu der Fadenaufnahme hin geführt sein und in dieser aufgenommen sein, solange der Stift von den Trägerarmen gehalten wird.

Gemäß einer weiteren Ausführungsform weist die Vorrichtung ferner eine Nadel auf, welche an einem freien Ende des Fadens befestigt sein kann.

Beispielsweise kann es beabsichtigt sein, zwei Fäden an einer Oberfläche eines Knochens zu befestigen. Hierzu kann ein Stift mit zwei Fäden in eine Bohrung eingesetzt werden, die vorab in dem Knochen ausgebildet wurde. Mit Hilfe von Ultraschallschwingungen kann nun das Material des Stiftes zumindest teilweise geschmolzen werden, sodass der Stift sicher in dem Knochen verankert wird. Auf diese Weise sind die zwei Fäden an dem Knochen verankert. Die Fäden können verwendet werden, um Weichgewebestrukturen an dem Knochen zu befestigen. Die Befestigung der Weichgewebestrukturen kann zusätzlich vereinfacht werden, wenn zumindest eine Nadel an einem freien Ende eines Fadens fixiert ist. Die Nadel kann nach einer Befestigung der Weichgewebestrukturen zusammen mit einer Überlänge des Fadens abgeschnitten und damit entfernt werden.

Alternative kann es beabsichtigt sein, einen oder mehrere Fäden an einem Objekt aus Stein, Steingut, Holz oder einem Kunststoff zu befestigen.

Die oben beschriebenen Aspekte und weitere Aspekte, Merkmale und Vorteile der Erfindung können ebenfalls aus den Beispielen der Ausführungsformen entnommen werden, welche im Folgenden unter Bezugnahme auf die anhängenden Zeichnungen beschrieben werden.

### KURZE BESCHREIBUNG DER ZEICHNUNGEN

Fig. 1 zeigt einen Träger mit einer Schutzkappe.
Fig. 2 zeigt einen Träger zusammen mit einem Stift mit Faden.
Fig. 3 zeigt einen Ultraschall-Applikator.
Fig. 4 zeigt einen Träger mit Schutzkappe an einem Ultraschall-Applikator.
Fig. 5 zeigt einen Träger mit Stift an einem Ultraschall-Applikator.
Fig. 6 zeigt, wie ein Stift in eine Bohrung eingesetzt wird.
Fig. 7 zeigt einen Träger mit einem in einer Bohrung verankerten Stift.
Fig. 8 zeigt eine in einer Bohrung verankerten Stift mit zwei Fäden.
Fig. 9 ist ein Flussdiagramm, welches Schritte eines Verfahrens zum Verankern eines Fadens an einem Objekt zeigt.

Es wird angemerkt, dass die Darstellungen in den Zeichnungen lediglich schematisch sind und keinen Hinweis geben sollen auf mögliche Größenverhältnisse. In den Figuren sind gleiche oder ähnliche Aspekte mit gleichen Bezugszeichen versehen.

### DETAILIERTE BESCHREIBUNG EINER AUSFÜHRUNGSFORM

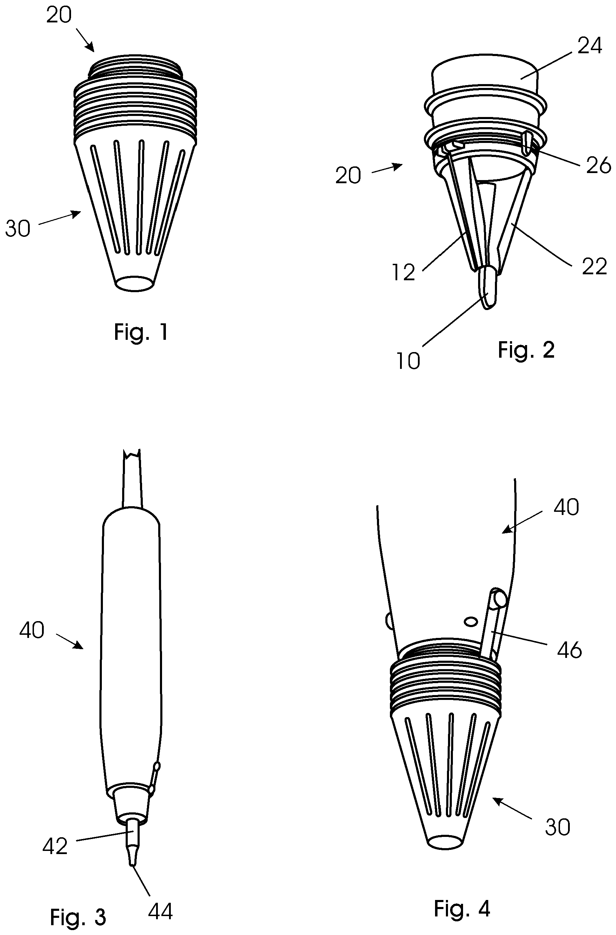

Figur 1 zeigt eine Schutzkappe 30, in welcher ein Träger 20 angeordnet ist. Die Schutzkappe 30 wird durch einen Hohlkörper gebildet, der einerseits einen ringförmigen Bereich und andererseits einen kegelstumpfförmigen Bereich aufweist. Die Außenflächen der Schutzkappe weisen Rillen oder Rippen auf, die ein Halten und Handhaben der Schutzkappe erleichtern. Die Schutzkappe 30 kann auch mit einer anderen äußeren Form ausgebildet sein, solange ein Hohlraum gebildet wird, der geeignet ist, einen Träger zusammen mit einem Stift aufzunehmen.

Figur 2 zeigt einen Träger 20, welcher einen ringförmigen Trägerkörper 24, zwei Trägerarme 22 und eine Fadenaufnahme 26 aufweist. Ein Stift 10, an dessen hinterem Ende zumindest ein Faden 12 befestigt ist, ist zwischen den Enden der Trägerarme 22 angeordnet und gehalten. Ein Faden 12 ist entlang eines Trägerarms 22 in Richtung des Trägerkörpers 24 geführt und in der Fadenaufnahme 26 aufgenommen. Die Fadenaufnahme 26 kann derart gestaltet sein, dass ein freies Ende eines Fadens eingeklemmt und damit gesichert werden kann. Darüber hinaus kann die Fadenaufnahme 26 so gestaltet sein, dass auch eine Nadel (nicht dargestellt) aufgenommen werden kann, welche am freien Ende des Fadens fixiert ist.

Figur 3 zeigt einen Ultraschall-Applikator 40, der an einem Ende einer Sonotrode 42 mit einer Sonotrodenspitze 44 aufweist. Die Sonotrode 42 steht von dem Gehäuse des Ultraschall-Applikators 40 aus vor. Innerhalb des Ultraschall-Applikators ist ein Ultraschall-Generator (nicht dargestellt) untergebracht, der mit der Sonotrode 42 verbunden ist, so dass Schwingungen, die von dem Ultraschall-Generator erzeugt werden, an die Sonotrode weitergegeben werden können.

In Figur 4 ist eine Kombination aus Träger 20 und Schutzkappe 30 an dem Ultraschall-Applikator 40 befestigt. An dem Ultraschall-Applikator ist ein Riegel 46 vorgesehen, der so ausgestaltet ist, dass mittels des Riegels 46 die Schutzkappe 30 wie auch der Träger an dem Gehäuse des Ultraschall-Applikators lösbar arretiert werden können. Beispielsweise kann der Riegel 46 derart betätigbar sein, dass die Schutzkappe 30, nicht jedoch der Träger 20 von dem Ultraschall-Applikator 40 abnehmbar ist.

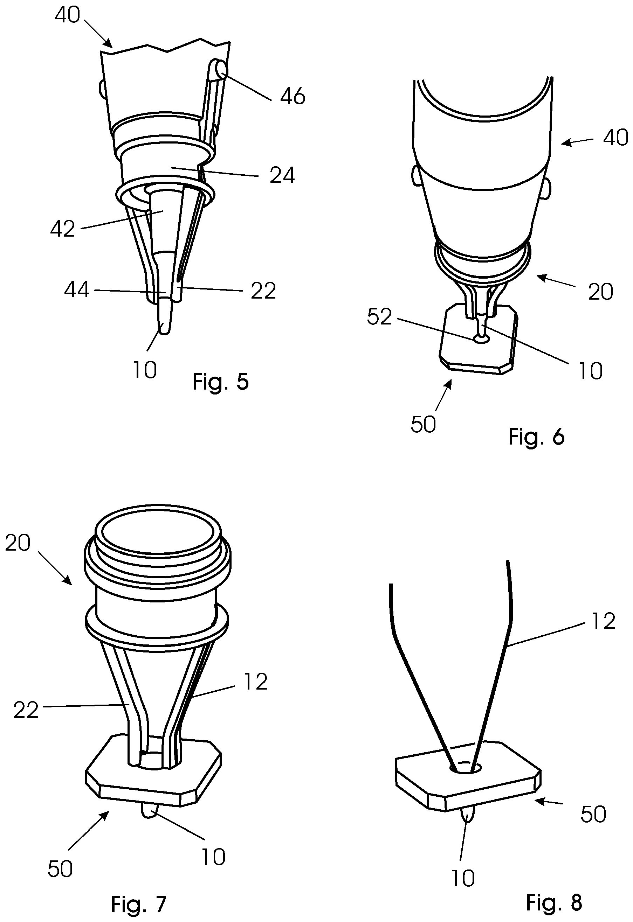

Figur 5 zeigt einen Zustand, in welchem ein Träger ohne Schutzkappe mit dem Ultraschall-Applikator 40 verbunden ist. In dieser Figur ist erkennbar, dass der Trägerkörper 24 durch den Riegel 46 an dem Ultraschall-Applikator arretierbar ist. Ferner ist zu sehen, dass die Trägerarme 22 den Stift 10 derart relativ zu dem Ultraschall-Applikator und damit zu der Sonotrode 42 halten, dass die Sonotrodenspitze 44 in Kontakt mit dem hinteren Ende des Stifts 10 ist. Dieser Kontakt ist notwendig, damit Schwingungen der Sonotrode auf das Material des Stiftes übertragen werden können.

Figur 6 zeigt eine Situation, in welcher der Stift 10 zu einer Öffnung 52 in einem Objekt 50 ausgerichtet gehalten wird. Die Öffnung 52 kann einer Bohrung oder eine geeignete Aussparung sein, wobei der Stift 10 einen Durchmesser oder eine Querschnitts-Form aufweist, die zu dem Durchmesser der Bohrung bzw. der Form der Aussparung passt.

Sobald der Stift 10 in die Öffnung 52 eingeführt oder eingesetzt ist, kann das Material des Stiftes durch Ultraschallschwingungen geschmolzen, zumindest weich gemacht werden, so dass das Material des Stifters 10 die Öffnung 52 inklusive der Oberflächenrauigkeiten ausfüllt, womit der Stift 10 fest in der Öffnung verankert wird.

An dieser Stelle wird darauf hingewiesen, dass die Öffnung 52 zum Beispiel auch eckig, sternförmig oder oval ausgebildet sein kann. Der Umstand, dass das Material des Stiftes 10 geschmolzen wird, macht es möglich, dass ein komplementär geformter Stift die Öffnung 52 ausfüllt, auch wenn die Querschnittsform des Stiftes nicht mit derjenigen der Öffnung übereinstimmt. Dieser Aspekt wird auch dadurch erleichtert, dass der Stift lediglich in die Öffnung eingesteckt wird, d.h., der Stift wird nur in Längsrichtung bzw. Einführrichtung bewegt, und muss nicht gedreht werden, wie beispielsweise einer Schraube.

Figur 7 zeigt einen Zustand, in welchem der Stift 10 bereits fest in dem Objekt 50 verankert ist und der Ultraschall-Applikator mit der Sonotrode entfernt wurde. In dieser Situation kann der Faden 12 in der Fadenaufnahme gelöst werden, so dass der Träger 20 von dem Stift und dem Faden getrennt werden kann.

Figur 8 zeigt einen Stift 10, der in einem Objekt 50 verankert ist, wobei an dem hinteren Ende des Stiftes 10 zwei Fäden 12 vorstehen. Hier ist zu erkennen, dass es möglich ist, Fäden derart an einem Objekt zu verankern, dass die Oberfläche des Objektes 50 bündig mit dem hinteren Ende eines Stifters sein kann.

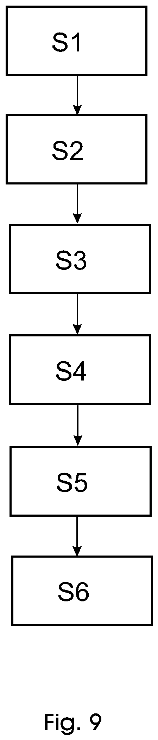

Das Flussdiagramm in Figur 9 illustriert Schritte eines Verfahrens zum Verankern eines Fadens an einem Objekt. Es wird angemerkt, dass die Schritte, die im Bezug auf das Verfahren und auch auf Figur 9 beschrieben werden, Haupt-Schritte sind, wobei diese Haupt-Schritte in Unter-Schritte differenziert und/oder unterteilt sein können. Außerdem können weitere Unter-Schritte zwischen den Haupt-Schritten sein. Ein Unter-Schritt ist jedoch nur genannt, wenn dieser Schritt wichtig für das Verständnis der Prinzipien des Verfahrens gemäß der Erfindung ist.

In Schritt S1 wird ein Stift bereitgestellt, an dessen hinterem Ende ein Faden befestigt ist, wobei der Stift aus einem Material hergestellt ist, welches mittels Ultraschallschwingungen verflüssigbar ist.

In Schritt S2 wird der Stift mit Hilfe eines Trägers gehalten. Mit anderen Worten, der Stift wird an dem Träger lösbar befestigt.

In Schritt S3 wird der Träger mit einem Ultraschall-Applikator verbunden, sodass eine Spitze einer Sonotrode des Ultraschall-Applikators in Kontakt mit dem Stift bringbar ist. Wenn zum Beispiel die Spitze der Sonotrode an einem vorderen Ende des Ultraschall-Applikators vorsteht, kann der Träger vorne auf den Ultraschall-Applikator aufgesteckt werden und gegebenenfalls arretiert werden.

In Schritt S4 wird der Stift in eine Bohrung in einem Objekt eingeführt. Das heißt, dass der Stift an dem Träger derart befestigt sein kann, dass die Spitze des Stiftes vorsteht. Auf diese Weise ist selbst ein sehr kleiner Stift handhabbar.

In Schritt S5 wird das Material des Stifts mit Hilfe von Ultraschallschwingungen, die von dem Ultraschall-Applikator erzeugt werden können, verflüssigt oder zumindest weich gemacht, sodass der Stift an dem Objekt verankert wird, zum Beispiel aufgrund eines Eindringens des Materials des Stiftes in Spalten und Poren in dem Objekt.

In Schritt S6 kann der Faden nach der Verankerung des Stifts an dem Objekt von einer Fadenaufnahme gelöst werden. Die Fadenaufnahme kann an dem Träger vorgesehen sein, sodass der Faden in Schritt S2 in der Fadenaufnahme aufgenommen werden kann und in den Schritten S3 bis S5 in dieser gehalten werden, sodass ein unbeabsichtigtes Verheddern und/oder Verknoten vermieden werden kann.

Während die Erfindung illustriert und beschrieben wurde im Detail in den Zeichnungen und der vorangegangenen Beschreibung, ist es beabsichtigt, dass derartige Illustrationen und Beschreibungen lediglich illustrativ oder exemplarisch und nicht restriktiv sind, so dass die Erfindung nicht durch die offenbarte Ausführungsform beschränkt ist. Andere Variationen der offenbarten Ausführungsform können durch einen Fachmann beim Umsetzen der beanspruchten Erfindung verstanden und bewirkt werden aus einem Studium der Zeichnungen, der Offenbarung und der anhängenden Ansprüche.

In den Ansprüchen schließt das Wort "aufweisend" nicht andere Elemente aus und der unbestimmte Artikel "ein" schließt eine Mehrzahl nicht aus.

Alleinig der Umstand, dass bestimmte Merkmale in verschiedenen abhängigen Ansprüchen genannt sind, beschränkt nicht den Gegenstand der Erfindung. Auch beliebige Kombinationen dieser Merkmale können vorteilhaft eingesetzt werden. Die Bezugszeichen in den Ansprüchen sollen nicht den Umfang der Ansprüche beschränken.

### BEZUGSZEICHENLISTE

- 10: Stift (pin)
- 12: Faden (suture)
- 20: Träger (shuttle)
- 22: Trägerarm (shuttle arm)
- 24: Trägerkörper (shuttle body)
- 26: Fadenaufnahme (suture reception)
- 30: Schutzkappe (protection cap)
- 40: Ultraschall-Applikator (ultrasonic applicator)
- 42: Sonotrode (sonotrode)
- 44: Sonotrodenspitze (tip of sonotrode)
- 46: Riegel (latch)
- 50: Objekt (object)
- 52: Öffnung (recess)

## Patentansprüche

1. Vorrichtung zum Verankern eines Fadens (12) an einem Objekt (50), aufweisend:
einen Ultraschall-Applikator (40) mit einer Sonotrode (42),
einen Stift (10) und
einen Träger (20),
wobei der Stift (10) aus einem Material hergestellt ist, welches mittels Ultraschallschwingungen verflüssigbar ist, und wobei der Faden (12) an seinem einen Ende unmittelbar mit dem Stift (10) fest verbindbar ist, und
wobei der Träger (20) einen Trägerkörper (24) und zumindest einen Trägerarm (22) aufweist, wobei der Trägerarm (22) geeignet ist, den Stift (10) in einer vorbestimmten Lage relativ zum Trägerkörper (24) lösbar zu halten und wobei der Trägerkörper (24) lösbar und derart mit dem Ultraschall-Applikator (40) verbunden ist, dass die Spitze (44) der Sonotrode (42) des Ultraschall-Applikators in Kontakt mit dem von dem Trägerarm (22) gehaltenen Stift (10) bringbar ist.

2. Vorrichtung gemäß Anspruch 1, wobei der Träger (20) ferner eine Fadenaufnahme (26) zum Aufnehmen des Fadens (12) aufweist.

3. Vorrichtung gemäß Anspruch 1 oder 2, ferner eine Schutzkappe (30) aufweisend, welche derart mit dem Träger (20) verbindbar ist, dass der von dem Trägerarm (22) gehaltene Stift (10) abgedeckt ist.

4. Vorrichtung gemäß einem der vorangegangenen Ansprüche, wobei der Ultraschall-Applikator (40) einen Riegel (46) zum Verriegeln des Trägers (20) an dem Ultraschall-Applikator (40) aufweist.

5. Vorrichtung gemäß einem der Ansprüche 2 bis 4,
wobei der Träger (20) zwei Trägerarme (22) aufweist, die derart angeordnet sind, dass der Stift (10) zwischen den zwei Trägerarmen (22) gehalten werden kann,
wobei die Fadenaufnahme (26) an dem Trägerkörper (24) vorgesehen ist, und wobei zwei Fäden (12) mit dem Stift (10) verbindbar sind, wobei jeweils ein Faden (12) entlang eines Trägerarms (22) zu der Fadenaufnahme (26) geführt und in dieser aufgenommen ist, wenn der Stift (10) von den Trägerarmen (22) gehalten wird.

6. Vorrichtung gemäß einem der vorangegangenen Ansprüche, ferner eine Nadel aufweisend, welche an einem freien Ende des Fadens befestigbar ist.

7. Verfahren zum Verankern eines Fadens an einem Objekt aus Stein, Steingut, Holz oder einem Kunststoff, die Schritte aufweisend:
- Bereitstellen eines Stiftes, an dessen hinterem Ende ein Faden unmittelbar befestigt ist, wobei der Stift aus einem Material hergestellt ist, welches mittels Ultraschallschwingungen verflüssigbar ist,
- Halten des Stifts mit Hilfe eines Trägers,
- Verbinden des Trägers mit einem Ultraschall-Applikator, sodass eine Spitze einer Sonotrode des Ultraschall-Applikators in Kontakt mit dem Stift bringbar ist,
- Einführen des Stifts in eine Bohrung in dem Objekt,
- Verflüssigen des Materials des Stifts mit Hilfe von Ultraschallschwingungen, die von dem Ultraschall-Applikator erzeugt werden können, sodass der Stift an dem Objekt verankert wird.

8. Verfahren nach Anspruch 7, wobei der Schritt des Haltens des Stiftes das Aufnehmen des Fadens an einer Fadenaufnahme des Trägers aufweist, und wobei der Faden nach der Verankerung des Stifts an dem Objekt von der Fadenaufnahme gelöst wird.

## Claims

1. Device for anchoring a suture (12) to an object (50), comprising:
an ultrasonic applicator (40) with a sonotrode (42),
a pin (10) and
a support (20),
wherein the pin (10) is produced from a material which can be liquefied by means of ultrasonic vibrations, and wherein the suture (12) is securely connected at one end thereof directly to the pin (10), and
wherein the support (20) comprises a support body (24) and at least one support arm (22), wherein the support arm (22) is configured for releasably holding the pin (10) in a predetermined position relative to the support body (24) und
wherein the support body (24) is releasably connected with the ultrasonic applicator (40) such that the tip (44) of the sonotrode (42) of the ultrasonic applicator is capable to be brought into contact with the pin (10) held by the support arm (22).

2. Device according to claim 1, wherein the support (20) further comprises a suture reception (26) for receiving the suture (12).

3. Device according to claim 1 or claim 2, further comprising a protection cap (30) which is capable to be connected to the support (20) such that the pin (10) held by the support arm (22) is covered.

4. Device according to any of the preceding claims, wherein the ultrasonic applicator (40) comprises a latch (46) for locking the support (20) at the ultrasonic applicator (40).

5. Device according to any of claim 2 to claim 4,
wherein the support (20) comprises two support arms (22) which are arranged such that the pin (10) can be held between the two support arms (22),
wherein the suture reception (26) is provided at the support body (24), and
wherein two sutures (12) are capable to be connected to the pin (10), wherein each suture (12) is guided along a support arm (22) towards the suture reception (26) and is received therein when the pin (10) is held by the support arms (22).

6. Device according to any of the preceding claims, further comprising a needle, which is capable to be fixed to a free end of the suture.

7. Method for anchoring a suture to an object of stone, stone, good, wood or a plastic, comprising the steps of:
- providing a pin, to the rear end of which a suture is directly fixed, wherein the pin is produced from a material which can be liquefied by means of ultrasonic vibrations,
- holding the pin by means of a support,
- connecting the support to an ultrasonic applicator such that a tip of a sonotrode of the ultrasonic applicator is capable to be brought into contact with the pin,
- introducing the pin into a hole in the object,
- liquefying the material of the pin by means of ultrasonic vibrations, which can be generated by the ultrasonic applicator such that the pin is anchored to the object.

8. Method according to claim 7, wherein the step of holding the pin comprises receiving the suture at a suture reception of the support, and wherein the suture is released from the suture reception after anchoring the pin to the object.

## Revendications

1. Dispositif destiné à ancrer un fil (12) à un objet (50), comportant :
un applicateur d'ultrasons (40) muni d'une sonotrode (42),
une pointe (10) et
un support (20),
dans lequel la pointe (10) est fabriquée à partir d'une matière qui peut être liquéfiée au moyen d'oscillations ultrasonores, et dans lequel le fil (12) peut être directement fixé à la pointe (10) au niveau de sa première extrémité, et
dans lequel le support (20) comporte un corps de support (24) et au moins un bras de support (22), dans lequel le bras de support (22) est adapté pour maintenir la pointe (10) dans une position prédéterminée par rapport au corps de support (24) de manière amovible, et dans lequel le corps de support (24) est amovible et relié à l'applicateur d'ultrasons (40) de telle sorte que l'embout (44) de la sonotrode (42) de l'applicateur d'ultrasons peut être mis en contact avec la pointe (10) maintenue par le bras de support (22).

2. Dispositif selon la revendication 1, dans lequel le support (22) comporte en outre un logement pour fil (26) destiné à recevoir le fil (12).

3. Dispositif selon la revendication 1 ou 2, comportant en outre un capuchon de protection (30) qui peut être relié au support (20) de manière à recouvrir la pointe (10) maintenue par le bras de support (22).

4. Dispositif selon l'une des revendications précédentes, dans lequel l'applicateur d'ultrasons (40) comporte un verrou (46) pour verrouiller le support (20) sur l'applicateur d'ultrasons (40).

5. Dispositif selon l'une des revendications 2 à 4,
dans lequel le support (20) comporte deux bras de support (22) qui sont disposés de telle sorte que la pointe (10) peut être maintenue entre les deux bras de support (22),
dans lequel le logement pour fil (26) est prévu sur le corps de support (24), et
dans lequel deux fils de suture (12) peuvent être reliés à la pointe (10), dans lequel un fil (12) est respectivement guidé le long d'un bras de support (22) jusqu'au logement pour fil (26) et est reçu dans celui-ci lorsque la pointe (10) est maintenue par les bras de support (22).

6. Dispositif selon l'une des revendications précédentes, comportant en outre une aiguille qui peut être fixée à une extrémité libre du fil.

7. Procédé pour ancrer un fil à un objet en pierre, faïence, bois ou matière plastique, comportant les étapes consistant à :
- fournir une pointe à l'extrémité arrière de laquelle est directement fixée un fil, la pointe étant fabriquée à partir d'une matière qui peut être liquéfiée au moyen d'oscillations ultrasonores,
- maintenir la pointe à l'aide d'un support,
- raccorder le support à un applicateur d'ultrasons de telle sorte qu'un embout d'une sonotrode de l'applicateur d'ultrasons peut être mise en contact avec la pointe,
- introduire la pointe dans un trou dans l'objet,
- liquéfier la matière de la pointe à l'aide d'oscillations ultrasonores qui peuvent être générées par l'applicateur d'ultrasons de telle sorte que la pointe est ancrée à l'objet.

8. Procédé selon la revendication 7, dans lequel l'étape de maintien de la pointe comporte la réception du fil sur un logement pour fil du support, et dans lequel le fil est libéré du logement pour fil après l'ancrage de la pointe à l'objet.
